# FASCICULE DE BREVET EUROPEEN

(11) **EP 3 463 487 B1**
(45) Date de publication et mention de la délivrance du brevet: **03.03.2021**
(21) Numéro de dépôt: 17729519.3
(22) Date de dépôt: 18.05.2017
(51) Int. Cl.: A61L 2/04, A61L 2/08, A61L 2/12, A61L 2/14, A61L 2/20, B67C 3/26, B67C 7/00

(54) **PROCEDE ET INSTALLATION DE FABRICATION ET DE TRAITEMENT DE RECIPIENTS**
VERFAHREN UND ANLAGE ZUR HERSTELLUNG UND BEHANDLUNG VON BEHÄLTERN
PROCESS AND PLANT FOR MANUFACTURING AND TREATING CONTAINERS

(30) Priorité: 25.05.2016 FR 1654683
(43) Date de publication de la demande: 10.04.2019
(73) Titulaire: Sidel Participations, 76930 Octeville-sur-Mer (FR)
(72) Inventeur: BERNARD, Véronique, 76930 Octeville-sur-mer (FR)
(74) Mandataire: Sidel Group
(86) Numéro de dépôt international: PCT/FR2017/051211
(87) Numéro de publication internationale: WO 2017/203136

(56) Documents cités:
- EP-A1- 2 279 952
- EP-A1- 2 394 950
- EP-A1- 2 866 844
- EP-A1- 2 866 844
- EP-A1- 3 069 846
- WO-A1-02/22447

## Description

### DOMAINE TECHNIQUE DE L'INVENTION

La présente invention concerne un procédé de traitement de corps creux et une installation de fabrication de récipients intégrant un tel procédé.

### ÉTAT DE LA TECHNIQUE

L'invention concerne plus particulièrement le traitement de corps creux en matière thermoplastique, tels que des bouchons ou des préformes, respectivement utilisés pour la fabrication de récipients dits « stériles » ou « ultra-propres ».

De tels récipients ultra-propres sont obtenus après la mise en œuvre d'une décontamination réalisée lors de la fabrication, notamment par voie chimique en utilisant un agent stérilisant comme le peroxyde d'hydrogène (H₂O₂).

Dans une installation de fabrication de récipients stériles ou ultra-propres en matière thermoplastique, notamment de bouteilles, il est en effet connu de mettre en œuvre différentes actions dans le but de contrôler et de maîtriser la qualité microbiologique de l'environnement de fabrication et tout particulièrement celle de l'intérieur des récipients.

On recherche d'une manière générale à éliminer tous les contaminants microbiens (micro-organismes), tels que des bactéries, des levures, des moisissures, etc.

De tels contaminants microbiens sont susceptibles d'affecter le produit conditionné dans les récipients, tout particulièrement dans le cas de produits agro-alimentaires.

Pour ce faire, on met avantageusement en œuvre un ensemble d'actions qui visent notamment à obtenir une décontamination des bouchons ainsi que des préformes à partir desquelles les récipients sont fabriqués.

Les documents de l'état de la technique cités ci-après et auxquels on se reportera pour de plus amples détails illustrent de manière non limitative des exemples d'actions visant plus particulièrement à éliminer les contaminants microbiens précités.

Le document WO-03/084818 décrit par exemple un traitement de décontamination par irradiation du col de préformes par un rayonnement de type ultraviolet (UV), avant l'introduction desdites préformes dans un four formant l'unité de chauffage de l'installation de fabrication de récipients.

Le document EP-2.094.312 décrit un autre exemple de traitement par irradiation avec un rayonnement ultraviolet (UV) qui est mis en œuvre de manière particulière dans un four pour décontaminer au moins la surface externe de la préforme.

Les documents WO-99/03667, EP2394950 et WO-2006/136498 décrivent des exemples de traitement de décontamination visant plus particulièrement l'intérieur d'une préforme, c'est à dire la paroi interne, traitée par voie chimique au moyen d'un agent stérilisant formé par du peroxyde d'hydrogène.

Le document WO-2006/136499 décrit plus précisément un procédé de décontamination de l'intérieur d'une préforme dans lequel l'agent stérilisant formé par du peroxyde d'hydrogène est déposé par condensation sur la paroi interne de la préforme, sous la forme d'un film de buée uniforme.

La préforme est ensuite conditionnée thermiquement dans un four afin d'être ramollie en vue de subir ensuite une transformation en récipient par soufflage ou par étirage-soufflage, le chauffage de la préforme ayant alors également pour fonction d'activer thermiquement le peroxyde d'hydrogène.

Au moins une partie du peroxyde d'hydrogène s'évapore alors sous l'effet du chauffage, passant de l'état liquide dans lequel il se trouve après avoir été déposé par condensation à l'état gazeux.

Néanmoins, des résidus de peroxyde d'hydrogène subsistent dans le récipient obtenu ensuite à partir d'une préforme ainsi décontaminée ce qui n'est pas sans poser différents problèmes détaillés ci-après.

Les résidus d'agent stérilisant, comme le peroxyde d'hydrogène, sont tout d'abord susceptibles de provoquer des altérations du produit conditionné dans le récipient, notamment par oxydation du produit.

Au nombre des altérations, on a par exemple pu constater selon les produits des changements de goût, de couleur ou encore une diminution de la teneur en vitamine C dans certains jus de fruit(s).

Or, certaines législations applicables dans le domaine agro-alimentaire imposent des valeurs maximales quant à la présence dans un récipient de résidus d'agent stérilisant comme le peroxyde d'hydrogène.

A titre d'exemple non limitatif, la FDA (Food and Drug Administration) aux Etats Unis prévoit, conformément à l'article 21 CFR Part 178.1005(d) du code « Fédéral Régulation » américain, que la concentration en peroxyde d'hydrogène dans un récipient utilisé pour le conditionnement d'un produit agro-alimentaire doit être inférieure à une concentration de 0,5 ppm.

Bien qu'un procédé de décontamination comme celui décrit dans le document WO-2006/136499 précité permette de réduire substantiellement la quantité de peroxyde d'hydrogène utilisée tout en conservant les degrés de décontamination obtenus précédemment, il n'en reste pas moins que des résidus sont toujours présents avec une certaine concentration dans le récipient final.

Le document EP 2866 844 décrit une méthode de stérilisation de bouteille en PET comprenant une étape d'introduction de peroxyde d'hydrogène dans la bouteille, une étape d'introduction d'air chaud dans la bouteille, et une étape d'introduction de plasma qui a lieu avant ou après l'étape d'introduction de peroxyde d'hydrogène.

De plus, une plus grande quantité de peroxyde d'hydrogène est utilisée pour la décontamination de récipients de faible contenance (inférieur à 1L), lesquels récipients ont par conséquent une concentration en résidus généralement supérieure, à proportion de leur volume. Or, les récipients de faible contenance ont connu un essor commercial important ces dernières années.

Par conséquent, on recherche des solutions permettant de réduire la concentration en résidus de peroxyde d'hydrogène dans les récipients obtenus à partir de préformes décontaminées chimiquement au moyen d'un tel agent stérilisant mais aussi dans les bouchons qui, utilisés pour les fermer, sont également décontaminés chimiquement.

Le but de l'invention est tout particulièrement de proposer une solution pour résoudre tout ou partie des problèmes décrits précédemment et notamment les problèmes de concentration en résidus de peroxyde d'hydrogène dans les récipients ultra-propres ou stériles.

### BREF RESUME DE L'INVENTION

Dans ce but, l'invention propose un procédé de traitement d'au moins un corps creux en matière thermoplastique, ledit procédé de traitement comportant successivement au moins les étapes suivantes :
(a) une première étape, consistant à traiter au moins l'intérieur du corps creux au moyen de peroxyde d'hydrogène ;
(b) une seconde étape, consistant à chauffer ledit corps creux ;
(c) une troisième étape, consistant à traiter le corps creux avec du plasma pour y réduire la concentration en résidus de peroxyde d'hydrogène.

Selon d'autres caractéristiques de l'invention :
- la première étape, de traitement, consiste à déposer par condensation un film de peroxyde d'hydrogène au moins à l'intérieur du corps creux en vue de sa décontamination ;
- la deuxième étape, de chauffage, consiste à chauffer le corps creux, dans lequel se trouve du peroxyde d'hydrogène, à une température déterminée qui est supérieure à la température d'activation du peroxyde d'hydrogène ;
- la deuxième étape, de chauffage du corps creux, est réalisée par des moyens de chauffage ;
- lors de la troisième étape, de traitement, le plasma est émis par au moins une buse qui est agencée de manière que le plasma seul pénètre à l'intérieur du corps creux ;
- l'extrémité libre de la buse d'injection du plasma est située à une distance du corps creux qui est inférieure à un centimètre, par exemple comprise entre 0,02 cm et 0,8 cm ;
- la durée d'application du plasma lors de la troisième étape, de traitement, est inférieure à une seconde, par exemple comprise entre 0,3 s et 0,6 s ;
- le corps creux est une préforme ;
- le corps creux est un bouchon ;

L'invention propose encore une installation de fabrication de récipients à partir de préformes en matière thermoplastique, ladite installation comportant au moins :
- une unité de traitement de l'intérieur au moins des préformes par application de peroxyde d'hydrogène ;
- une unité de chauffage des préformes ;
- une unité de moulage de récipients formés par soufflage ou par étirage-soufflage à partir des préformes chaudes ;
caractérisée en ce que l'installation comporte au moins une unité de traitement par plasma des préformes décontaminées qui est agencée en amont de l'unité de moulage pour réduire la concentration en résidus de peroxyde d'hydrogène à l'intérieur des préformes.

Avantageusement, l'unité de traitement par application de peroxyde d'hydrogène est agencée en amont de l'unité de chauffage des préformes et l'unité de traitement par plasma est agencée entre l'unité de chauffage et l'unité de moulage, afin que le traitement des préformes par plasma soit effectué après qu'elles aient été préalablement décontaminées par le peroxyde d'hydrogène dans l'unité de traitement correspondante.

### BREVE DESCRIPTION DES FIGURES

D'autres caractéristiques et avantages de l'invention apparaitront au cours de la lecture de la description détaillée qui va suivre pour la compréhension de laquelle on se reportera aux dessins annexés dans lesquels :

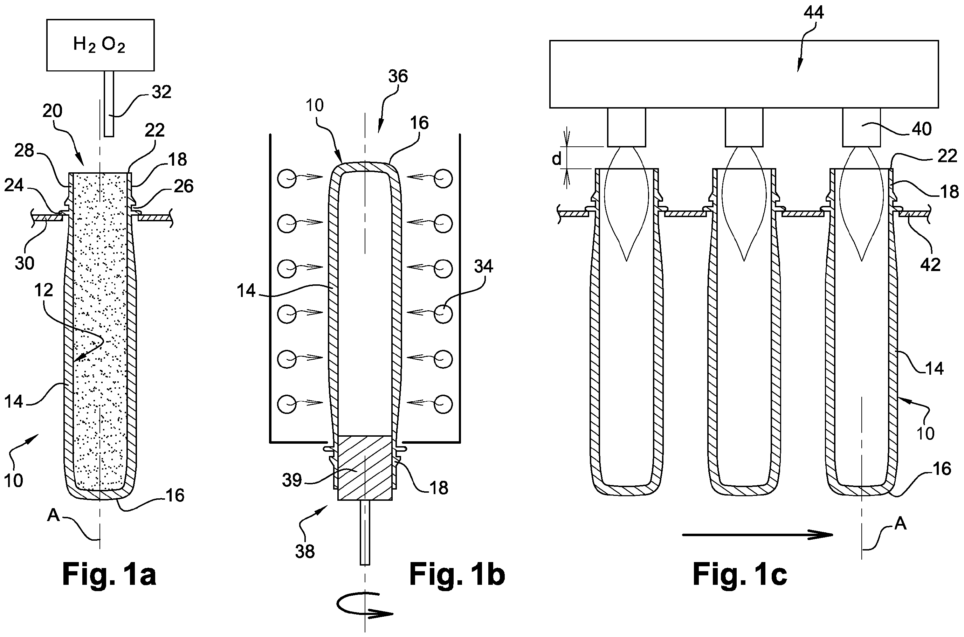

- les figures 1a, 1b et 1c sont des vues qui représentent schématiquement les étapes du procédé de traitement selon l'invention pour traiter au moins une préforme en matière thermoplastique destinée à la fabrication d'un récipient et qui illustrent successivement le traitement de l'intérieur au moins de la préforme avec du peroxyde d'hydrogène en vue de sa décontamination, le chauffage du corps de la préforme et un traitement par plasma d'au moins une partie de l'intérieur de la préforme afin d'y réduire la concentration en résidus de peroxyde d'hydrogène ;

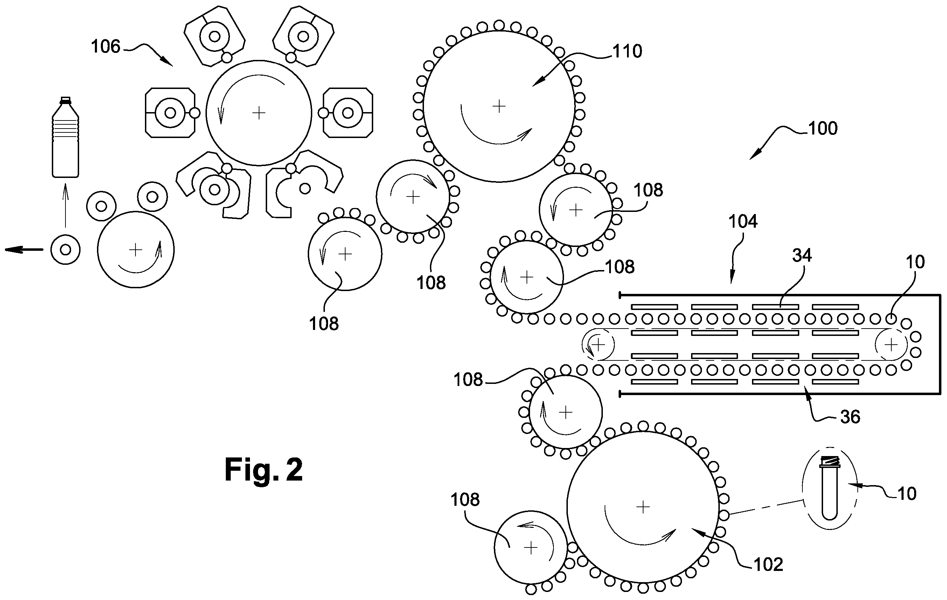

- la figure 2 est une vue schématique qui représente un exemple de réalisation d'une installation de fabrication de récipients ultra-propres et qui illustre une unité de traitement par plasma des préformes intégrée à l'installation afin d'y mettre en œuvre le procédé de traitement selon l'invention ;

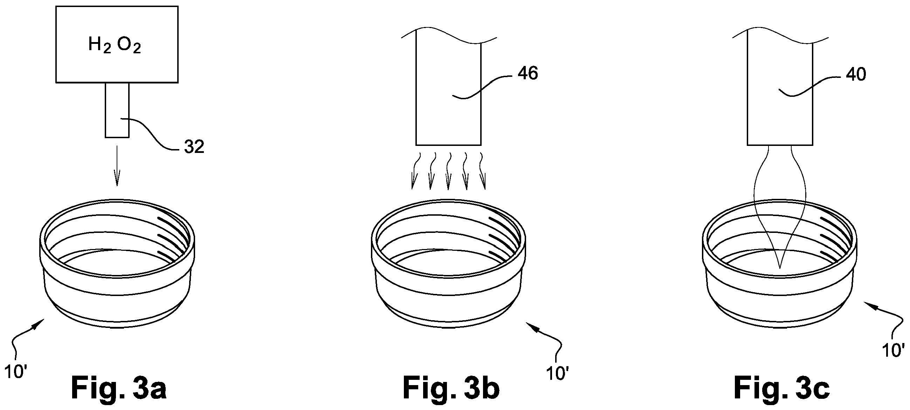

- les figures 3a, 3b et 3c sont des vues qui représentent schématiquement les étapes du procédé de traitement d'au moins un bouchon en matière thermoplastique destiné à fermer un récipient et qui illustrent successivement le traitement de l'intérieur du bouchon avec du peroxyde d'hydrogène en vue de sa décontamination, le chauffage à l'air chaud du bouchon et un traitement par plasma de l'intérieur du bouchon afin d'y réduire la concentration en résidus de peroxyde d'hydrogène.

### DESCRIPTION DETAILLEE DES FIGURES

On a représenté sur les figures 1a à 1c, un corps creux formé par une préforme 10 en matière thermoplastique pour illustrer un premier exemple d'application du procédé de traitement selon l'invention.

La préforme 10 est obtenue par moulage par injection de matière plastique et présente des caractéristiques (dimensions, répartition de la matière, etc.) qui sont déterminées par le récipient final à obtenir, en particulier sa forme ou encore sa contenance.

Une telle préforme 10 est utilisée pour la fabrication de différents types de récipients (bouteilles, flacons, pots, etc.) qui sont notamment, mais non exclusivement, destinés à être utilisés pour le conditionnement de produits agro-alimentaires.

La préforme 10 comporte une surface 12 interne délimitée par un corps 14 fermé à une extrémité par un fond 16 et qui, à l'autre extrémité, comporte un col 18 délimitant une ouverture 20, en général circulaire, d'accès à l'intérieur de la préforme 10.

La préforme 10 présente un axe A principal qui s'étend axialement du fond 16 jusqu'au col 18.

Le col 18 de la préforme 10 présente sa forme définitive à l'issue de la fabrication par injection de la préforme et correspond au col du récipient final, un bord 22 du col (ou buvant) délimitant la circonférence de l'ouverture 20 qui constitue le seul accès à l'intérieur de la préforme 10.

Le diamètre interne de la préforme 10 peut varier sur la hauteur suivant l'axe A principal de la préforme, c'est à dire entre le col 18 et le fond 16.

Dans l'exemple représenté aux figures 1a à 1c, le col 18 comporte une collerette 24 qui s'étend radialement en saillie vers l'extérieur et une gorge 26 annulaire qui, adjacente à ladite collerette 24 est destinée à recevoir ultérieurement une bague d'inviolabilité.

Une telle bague est destinée à garantir au consommateur l'intégrité du récipient final rempli avant une première ouverture.

De préférence, le col 18 comporte un filetage 28 destiné à permettre la fermeture du récipient final par un bouchon à vis complémentaire.

Pour son transport, la préforme 10 est susceptible d'être prise par l'intérieur du col 18 par l'intermédiaire de moyens de préhension (c'est notamment le cas dans les fours de chauffage où les préformes sont portées par des mandrins insérés dans l'ouverture du col18) mais la collerette 24 de la préforme 10 ou la gorge 26 annulaire sont également souvent utilisées.

Tel qu'illustré sur la figure 1a, la préforme 10 est supportée par des moyens 30 de transport qui coopèrent avec une partie d'une face inférieure de la collerette 24.

De manière connue, les moyens 30 de transport sont par exemple constitués par des rails ou encore un plateau muni d'encoches appartenant à une roue de transfert ou encore des bras équipés de pinces de préhension.

Comme expliqué en préambule, on recherche des solutions pour fabriquer des récipients ultra-propres (ou stériles) comportant une concentration réduite en peroxyde d'hydrogène.

La présence de peroxyde d'hydrogène dans les récipients résulte de l'utilisation de peroxyde d'hydrogène comme agent stérilisant pour décontaminer d'une part les bouchons et, d'autre part, au moins l'intérieur des préformes qui sont traitées préalablement à leur transformation en récipients.

L'invention propose un procédé de traitement d'au moins un corps creux en matière thermoplastique, tel qu'une préforme ou un bouchon, pour réduire la concentration en peroxyde d'hydrogène dans un récipient final, c'est à dire fermé par un bouchon.

Le procédé de traitement du corps creux en matière thermoplastique comporte successivement au moins les étapes suivantes :
(a) une première étape, consistant à traiter au moins l'intérieur du corps creux au moyen de peroxyde d'hydrogène ;
(b) une seconde étape, consistant à chauffer ledit corps creux ;
(c) une troisième étape, consistant à traiter le corps creux avec du plasma pour y réduire la concentration en résidus de peroxyde d'hydrogène.

Ainsi, le procédé de traitement selon l'invention comporte au moins une étape de décontamination d'au moins l'intérieur du corps creux au moyen de peroxyde d'hydrogène.

Dans l'exemple d'application représenté sur les figures 1a, 1b et 1c, le corps creux est constitué par une préforme 10 en matière thermoplastique, telle que du PET.

La préforme 10 est destinée à être transformée en un récipient par soufflage ou par étirage-soufflage dans un moule après avoir été préalablement conditionnée thermiquement afin d'en ramollir la matière thermoplastique.

Dans ce premier exemple d'application, on procède à une décontamination d'au moins l'intérieur de la préforme 10 grâce à du peroxyde d'hydrogène, c'est-à-dire à la décontamination de l'ensemble de la surface 12 interne destinée à être ultérieurement en contact avec le produit conditionné une fois le récipient formé.

L'étape de décontamination dudit procédé de traitement comporte au moins une première étape (a), de traitement avec du peroxyde d'hydrogène, et une deuxième étape (b), de chauffage de la préforme 10.

De préférence, comme illustré à la figure 1a, la première étape (a), de traitement en vue de la décontamination, consiste à déposer par condensation du peroxyde d'hydrogène au moins à l'intérieur de la préforme 10.

Le peroxyde d'hydrogène est introduit à l'intérieur de la préforme 10 sous forme d'un mélange gazeux comportant également de l'air chaud. Le peroxyde d'hydrogène à l'état liquide est tout d'abord évaporé puis mélangé à de l'air chaud pour être acheminé jusqu'à la préforme 10 à traiter.

Lorsque ledit mélange chaud comportant le peroxyde d'hydrogène entre en contact avec la surface 12 interne d'une préforme 10 présentant une température inférieure (par exemple à température ambiante), un phénomène de condensation va alors se produire et aboutir au dépôt d'un film de buée uniforme sur l'ensemble de la surface 12 interne.

De manière non limitative, on a matérialisé par une multitude de points le film de buée de peroxyde d'hydrogène déposé par condensation sur l'ensemble de la surface 12 interne de la préforme 10.

On se reportera avantageusement aux documents précités WO-2006/136498 et surtout WO-2006/136499 pour de plus amples détails sur la décontamination d'une préforme 10 selon ce procédé de décontamination de préformes.

Ce procédé de décontamination est par ailleurs exploité commercialement par la Demanderesse sous la désignation « Prédis™ » (marque déposée).

Sur la figure 1a, on a représenté de manière non limitative une seule préforme 10 pour illustrer simplement l'étape (a).

L'étape (a), de traitement, est toutefois susceptible d'être réalisée simultanément ou quasi simultanément dans une ou plusieurs préformes respectivement traitées en restant fixes ou en étant en mouvement relativement à des moyens d'injection du peroxyde d'hydrogène. Comme illustré sur la figure 1a, les moyens d'injection du peroxyde d'hydrogène comportent au moins une buse 32 d'injection.

De préférence, la préforme 10 occupe une position fixe relativement à la buse 32 d'injection du peroxyde d'hydrogène qui est introduit axialement à l'intérieur de la préforme 10 par l'ouverture 20 délimitée radialement par le bord 22 du col 18.

Avantageusement, la buse 32 d'injection est décalée radialement par rapport à l'axe A de la préforme 10 de sorte que l'axe principal de la buse 32 ne soit pas coaxial à l'axe A de la préforme 10.

En variante non représentée, le peroxyde d'hydrogène est injecté dans la préforme 10 par plus d'une buse 32 d'injection. Le peroxyde d'hydrogène est par exemple injecté au défilé, successivement par plusieurs buses adjacentes par rapport auxquelles l'ouverture 20 de la préforme 10 subit un déplacement relatif. En d'autres termes, les préformes 10 peuvent être déplacées devant la ou les buses et/ou vice versa.

La deuxième étape (b), de chauffage, consiste à chauffer le corps creux constitué par la préforme 10.

L'étape (b), de chauffage, a au moins pour fonction d'activer thermiquement le peroxyde d'hydrogène préalablement injecté lors de l'étape (a) afin d'obtenir l'effet de décontamination.

L'étape (b), de chauffage, consiste à chauffer la préforme 10 à une température qui est supérieure à la température d'activation du peroxyde d'hydrogène.

La température d'activation du peroxyde d'hydrogène est d'environ soixante-dix degrés Celsius (70°C).

Le peroxyde d'hydrogène déposé par condensation sur la surface 12 interne de la préforme 10 forme avantageusement un film de buée uniforme et se trouve par conséquent au moins en partie à l'état liquide.

Le chauffage selon l'étape (b) provoque une évaporation d'au moins une partie du film, dans lequel la concentration en peroxyde d'hydrogène va ainsi croître, augmentant alors l'effet décontaminant obtenu.

De préférence, lorsque le corps creux est une préforme 10, la deuxième étape (b), de chauffage, a également pour fonction de chauffer le corps 14 de la préforme 10 à une température de moulage pour permettre sa transformation ultérieure en récipient.

Bien que la température de moulage varie en fonction des préformes 10, la température de moulage est généralement comprise entre quatre-vingt-quinze degrés Celsius (95°C) et cent trente-cinq degrés Celsius (135°C).

La température de moulage de la préforme 10 est donc supérieure à la température d'activation du peroxyde d'hydrogène.

La deuxième étape (b), de chauffage de la préforme 10, est réalisée par des moyens 34 de chauffage.

Les moyens 34 de chauffage sont par exemple des moyens de chauffage à rayonnement infrarouge (IR) ou des moyens de chauffage de type laser ou encore par micro-ondes. En variante, les moyens 34 de chauffage sont des moyens de chauffage à l'air chaud.

Dans le cas de préformes 10, l'étape (b), de chauffage, est réalisée dans au moins un four 36 qui est muni de tels moyens 34 de chauffage.

De préférence, le chauffage du corps 14 de la préforme 10 est réalisé dans une position dite « col en bas » afin d'éviter de chauffer le col 18 par convection.

De manière connue, le col 18 présente sa forme définitive et ne doit pas être chauffé contrairement au corps 14, le four 36 comporte avantageusement des moyens de refroidissement (non représentés) du col 18 des préformes 10. Les moyens de refroidissement sont par exemple constitués par un flux d'air filtré qui est mis en circulation dans le four 36 pour refroidir au moins les cols 18 des préformes 10.

Dans un four 36, les préformes 10 sont transportées par un dispositif 38 de transport comportant des moyens 39 de préhension de chaque préforme 10, par exemple par l'intérieur du col 18.

Avantageusement, les moyens 39 de préhension sont aptes à entraîner également chaque préforme 10 en rotation sur elle-même autour de son axe A, simultanément au déplacement à travers le four 36 suivant un parcours de chauffe donné.

Un tel dispositif 38 de transport de préformes dans un four et des moyens 39 de préhension de préformes sont par exemple décrits dans le document WO-A-00/48819 auquel on se reportera pour de plus amples détails.

De la même manière, on pourra se reporter à titre d'exemples non limitatifs de fours de chauffage de préformes aux documents EP-A-0.620.099 et EP-A-0.564.354.

La troisième étape (c), de traitement, du procédé selon l'invention consiste à utiliser un plasma pour réduire la concentration de peroxyde d'hydrogène présente à l'intérieur de la préforme 10 à l'issue de l'étape de décontamination préalablement mise en œuvre.

Conformément à l'invention, lors de la troisième étape (c), de traitement, un plasma est injecté par au moins une buse 40 d'injection de façon à être introduit à l'intérieur de la préforme 10.

De préférence, la buse 40 d'injection du plasma est agencée de manière que le plasma seul pénètre à l'intérieur du corps creux formé, dans ce premier exemple, par la préforme 10.

En variante non représentée, la buse 40 d'injection du plasma pénètre à l'intérieur du corps creux formé, dans ce premier exemple par la préforme 10, pour y introduire le plasma.

Avantageusement, l'extrémité libre de la buse 40 d'injection du plasma est située à une distance « d » du bord 22 du col 18 de la préforme 10 qui est inférieure à un centimètre (1 cm). La distance « d » entre le bord 22 du col 18 de la préforme 10 et l'extrémité de la buse 40 est par exemple comprise entre 0,02 cm et 0,8 cm.

Tel qu'illustré sur la figure 1c, l'étape (c), de traitement, est réalisée simultanément dans plusieurs préformes 10 qui sont ainsi traitées au défilé par une série de buses 40 agencées les unes à côté des autres sur un parcours.

De préférence, les préformes 10 sont déplacées relativement aux buses 40 par un dispositif 42 de transport, tel qu'un plateau monté mobile en rotation, suivant la flèche représentée sur la figure 1c.

La durée d'application du plasma lors de la troisième étape (c), de traitement, est inférieure à une seconde. De préférence, la durée d'application du plasma lors de cette troisième étape (c) est par exemple comprise entre 0,3 s et 0,6 s.

Grâce à la troisième étape (c), de traitement par plasma, on élimine au moins une partie du peroxyde d'hydrogène subsistant à l'intérieur de la préforme 10 après l'opération de décontamination des étapes (a) et (b).

Le plasma permet après réaction de décomposer le peroxyde d'hydrogène (H₂O₂) en deux composants inoffensifs, respectivement de l'eau (H₂O) et du dioxygène (O₂) :

**2 H₂O₂ → 2 H₂O + O₂**

La durée de traitement par plasma pour réduire la quantité résiduelle de peroxyde d'hydrogène de façon significative est avantageusement inférieure à une seconde (1 s).

L'étape (c), de traitement par plasma, permet de réduire au moins par deux la concentration en peroxyde d'hydrogène dans le récipient obtenu à partir d'une préforme 10.

Le plasma introduit par l'ouverture 20 du col 18 va notamment agir sur le peroxyde d'hydrogène présent sur la surface 12 interne au niveau du col 18 de la préforme 10.

Or la concentration en peroxyde d'hydrogène au niveau du col 18 est généralement plus importante que sur le reste de la surface 12 interne de la préforme 10, notamment au niveau de son corps 14.

Cela résulte du fait que, lors de l'étape (b), de chauffage, les cols 18 des préformes 10 ne sont pas chauffés dans le four 36 contrairement aux corps 14.

Lors de la troisième étape (c), le plasma introduit à l'intérieur de la préforme 10 peut par conséquent ne traiter qu'une partie de la surface 12 interne de la préforme 10 étant rappelé que la concentration en résidus de peroxyde d'hydrogène est considérée sur l'ensemble du récipient obtenu à partir d'une telle préforme 10.

Tel que représenté schématiquement sur la figure 1c, le plasma est par exemple produit par un dispositif 44 qui est apte à alimenter en plasma une ou plusieurs buses 40.

De préférence, le plasma est du type « atmosphérique » c'est-à-dire dont le jet est projeté à l'air libre.

Le plasma est obtenu au moyen d'une décharge électrique et d'un gaz qui est avantageusement constitué au moins par de l'air. Le choix de l'air présente des avantages, notamment un coût réduit en raison de sa grande disponibilité.

A titre d'exemple non limitatif, un générateur de plasma froid à pression atmosphérique est commercialisé par la société PlasmaTreat® sous la désignation « OPENAIR™».

Avantageusement, un tel plasma froid à pression atmosphérique est homogène, c'est-à-dire continu dans le temps et régulier sur la surface, sans potentiel (la torche étant reliée à la terre on obtient un jet de plasma hors tension électrique), et de forte intensité.

De préférence, le plasma utilisé est obtenu à partir d'air, en variante à partir d'un mélange de gaz constitué au moins en partie d'air.

En variante, le plasma est obtenu à partir d'au moins un autre gaz que l'air (ou un mélange de gaz), avantageusement un gaz inerte, tel que de l'azote, de l'argon ou de l'hélium.

Le type de buse 40 utilisée pour projeter le plasma est susceptible de varier en fonction notamment du type de corps creux traités, tout particulièrement dans ce premier exemple d'application du type de préformes 10.

Le choix de la buse 40 de projection du plasma est également déterminé selon que le plasma est appliqué ou non depuis l'extérieur de la préforme 10 ou encore selon que la préforme 10 est ou non en mouvement relativement à au moins une buse 40.

Dans un mode alternatif, on utilise plusieurs types de buses pour traiter une même préforme 10 de façon à traiter plus spécifiquement certaines zones de la surface 12 interne de la préforme, par exemple une buse pour traiter spécifiquement le fond 16 de la préforme 10 et/ou une buse pour traiter spécifiquement une partie du corps 14 de la préforme 10 avec introduction ou non de la buse dans la préforme 10.

De nombreuses formes géométriques de buse existent parmi lesquelles figurent par exemple des buses allongées présentant globalement une forme de doigt typiquement adaptée à être introduite à l'intérieur d'un corps creux comme une préforme 10 présentant un col 18 avec une ouverture 20 réduite.

On distingue également les buses « fixes » des buses rotatives qui sont susceptibles d'être entraînées en rotation sur elle-même.

Tel qu'indiqué précédemment, de préférence, la buse 40 délivrant le plasma n'est pas introduite à travers l'ouverture 20 du col 18 de la préforme 10 lors de la troisième étape (c), de traitement, et ceci afin de conserver une durée de traitement avantageusement brève, notamment compatible avec les cadences de fabrication de récipients.

En bref, pour qu'il y ait réduction du résidu de peroxyde d'hydrogène présent sur la surface 12 interne du corps creux, il est nécessaire que le plasma entre en contact avec le résidu de peroxyde d'hydrogène.

La longueur du plasma entre la sortie de la buse et l'extrémité du plasma est au maximum de 5 centimètre. Dès lors, il est compréhensible que l'utilisation de plasma dans le cadre d'une bouteille n'ait pas satisfaisant parce que les dimensions de la bouteille et du plasma ne sont pas compatible. En effet, pour réaliser le traitement, il serait nécessaire de déplacer le plasma dans la bouteille et cela au détriment de la durée d'application du plasma lors de la troisième étape (c) de traitement par plasma qui est inférieure à une seconde (1 s), par exemple comprise entre 0,3 s et 0,6 s.

De plus, la forme du plasma peut être comparée à une flamme. En conséquence cette forme est d'autant plus compatible pour traiter l'intérieur d'une préforme que pour traiter l'intérieur d'une bouteille. En effet, dans le cas d'une préforme, le volume interne de la préforme correspond sensiblement à un cylindre dont le diamètre est compris entre 10 et 40 millimètre. Par conséquent, le plasma lèche la paroi interne de la préforme alors que dans le cas d'une bouteille, le plasma ne peut pas venir lécher la paroi interne de la bouteille sur toute la longueur et/ou sur tout le pourtour.

On a représenté à la figure 2, un exemple de réalisation d'une installation 100 de fabrication de récipients à partir de préformes 10 en matière thermoplastique.

La figure 2 illustre plus précisément une installation 100 dans laquelle le procédé de traitement qui vient d'être décrit en référence à la figure 1 serait mis en œuvre pour traiter des préformes 10.

L'installation 100 de fabrication comporte au moins une unité 102 de traitement de l'intérieur au moins des préformes 10 par application de peroxyde d'hydrogène, une unité 104 de chauffage des préformes 10, une unité 106 de moulage de récipients à partir des préformes 10 chaudes.

L'unité 102 de traitement est par exemple apte à traiter des préformes 10 en introduisant à l'intérieur d'au moins une préforme 10 du peroxyde d'hydrogène pour obtenir un dépôt par condensation d'un film de peroxyde d'hydrogène sur la surface 12 interne de la préforme 10.

L'unité 102 de traitement permet la mise en œuvre de la première étape (a), de traitement selon le procédé, telle que décrite précédemment en référence à la figure 1a.

L'unité 104 de chauffage comporte au moins un four 36 pourvu de moyens 34 de chauffage pour la mise en œuvre de l'étape (b), de chauffage, selon le procédé de l'invention et le conditionnement thermique des corps 14 des préformes 10 en vue de leur transformation en récipients.

En variante non représentée, les unités 102 et 104 sont une seule et même unité, c'est-à-dire que l'injection de peroxyde d'hydrogène est réalisée dans l'unité 104 de chauffe.

L'unité 106 de moulage de récipients (encore appelée « souffleuse ») est destinée à transformer en récipients les préformes 10 chaudes issues de l'unité 104 de chauffage par soufflage ou par étirage-soufflage qui est réalisé dans un moule.

Les différentes unités 102, 104 et 106 sont notamment reliées entre elles par des dispositifs 108 de transport, tels que des roues de transfert.

L'installation 100 comporte au moins une unité 110 de traitement par plasma des préformes 10 préalablement décontaminées avec du peroxyde d'hydrogène afin de réduire la concentration en résidus de peroxyde d'hydrogène dans les préformes 10 et par conséquent dans les récipients obtenus à partir de telles préformes 10.

L'unité 110 de traitement par plasma est agencée en amont de l'unité de moulage pour réduire la concentration en résidus de peroxyde d'hydrogène stérilisant à l'intérieur des préformes 10.

L'unité 110 de traitement par plasma correspond à la mise en œuvre de l'étape (c), de traitement, selon le procédé décrit en référence à la figure 1c.

L'étape (c), de traitement par plasma, est avantageusement susceptible d'être mise en œuvre dans une installation 100 de fabrication de récipients sans en affecter les cadences de production par rapport aux unités connues jusqu'alors (par exemple exprimées en nombre de bouteilles par heure).

En effet, la durée de traitement par plasma étant inférieure à une seconde, il est possible d'intégrer une telle unité 110 dans le parcours de transfert suivi par le flux de préformes 10 à travers l'installation 100, plus précisément entre la sortie de l'unité 104 de chauffage et l'unité 106 de moulage.

Tel que représenté sur la figure 2, l'unité 110 de traitement par plasma est agencée dans l'installation 100 entre l'unité 104 de chauffage et l'unité 106 de moulage, qui permet ainsi de fabriquer des récipients à partir de préformes 10 préalablement décontaminées avec du peroxyde d'hydrogène grâce à l'unité 102 de traitement qui, elle, est agencée en amont de l'unité 104 de chauffage des préformes.

Le fait de traiter la préforme par plasma entre l'unité de chauffage des préformes et l'unité de moulage de récipients provoque un effet surprenant qui est de permettre la réduction de la majeur partie du résidu de peroxyde d'hydrogène. Cette forte réduction du résidu de peroxyde d'hydrogène n'a pas lieu si le traitement par plasma est réalisé après l'unité de moulage par soufflage sous pression. L'inventeur s'est rendu compte que le soufflage sous pression provoque l'absorption d'une partie du résidu de peroxyde d'hydrogène par le corps creux en matière thermoplastique du futur récipient et l'autre partie du résidu reste en surface. Dès lors, l'expérience a montré que le traitement plasma réalisé sur la bouteille ne réduit que le résidu de peroxyde d'hydrogène restant en surface, et n'a pas d'effet sur le peroxyde d'hydrogène absorbé par le matériau du futur récipient.

Dans le cas d'un traitement plasma réalisé en sortie de l'unité de chauffage, des mesures de résiduel de peroxyde d'hydrogène ont été effectuées vingt-quatre heures après le traitement plasma. Les résultats montrent que la quantité de résiduel de peroxyde d'hydrogène a diminué de cinquante pourcents.

Dans le cas d'un traitement plasma réalisé en sortie de l'unité de moulage, des mesures de peroxyde d'hydrogène ont été effectuées également vingt-quatre heures après le traitement plasma. Les résultats montrent que la quantité de résiduel de peroxyde d'hydrogène à non pas diminuée, mais a augmentée de deux cent pourcents. Cela s'explique par le fait que la quantité de peroxyde d'hydrogène absorbée par le matériau se relargue à la surface interne du récipient.

On a représenté aux figures 3a, 3b et 3c, un deuxième exemple d'application du procédé de traitement selon l'invention.

Dans ce deuxième exemple, le corps creux est un bouchon 10' en matière thermoplastique.

Un tel bouchon 10' est destiné à coopérer, notamment par vissage, avec un col d'un récipient afin d'en permettre la fermeture.

Le procédé de traitement d'au moins un bouchon 10' comporte successivement au moins les étapes suivantes :
(a) une première étape, consistant à traiter au moins l'intérieur du bouchon 10' au moyen de peroxyde d'hydrogène ;
(b) une seconde étape, consistant à chauffer ledit bouchon 10' ;
(c) une troisième étape, consistant à traiter le bouchon 10' avec du plasma pour y réduire la concentration en résidus de peroxyde d'hydrogène.

De préférence, le bouchon 10' est traité avec du peroxyde d'hydrogène de la même manière que la préforme 10 selon la figure 1a, à savoir qu'un film de buée de peroxyde d'hydrogène est déposé par condensation à l'intérieur du bouchon 10'.

Tel que représenté avec la première étape (a) de la figure 3a, le mélange gazeux comprenant le peroxyde d'hydrogène est projeté par une buse 32 d'injection en direction de l'intérieur du bouchon 10'.

La deuxième étape (b), de chauffage du bouchon 10', est destinée à activer thermiquement le peroxyde d'hydrogène présent afin d'obtenir la décontamination de l'intérieur du bouchon 10'.

De préférence, la deuxième étape (b), de chauffage, est réalisée avec des moyens 46 de chauffage formés par de l'air chaud.

En variante, on pourrait également utiliser d'autres moyens de chauffage équivalents, comme par exemple des moyens de chauffage à rayonnement infrarouge, de type laser ou encore par micro-ondes.

Dans l'exemple illustré sur la figure 3b, les moyens 46 de chauffage pour obtenir l'air chaud comportent par exemple des résistances électriques chauffantes et des moyens de ventilation pour projeter l'air chaud en direction du ou des bouchons 10'.

L'air chaud délivré par les moyens 46 de chauffage présente une température qui est supérieure à la température d'activation thermique du peroxyde d'hydrogène
Une fois l'étape de décontamination du bouchon 10' réalisée, la troisième étape (c) consiste, comme précédemment pour la préforme 10, à appliquer un plasma délivré par une buse 40 pour traiter le bouchon 10' afin de réduire la concentration en résidus de peroxyde d'hydrogène présents sur le bouchon 10'.

Bien entendu la représentation faite sur les figures 3a à 3c n'est nullement limitative et plusieurs bouchons 10' sont susceptibles d'être traités, simultanément ou non, notamment au défilé au moyen d'une pluralité de buses 40 pour l'application du plasma par exemple.

Pour la troisième étape (c), de traitement du bouchon 10', les paramètres sont avantageusement similaires à ceux décrits précédemment pour la préforme 10.

De préférence, le plasma est émis par la ou les buses 40 qui est ou sont agencées au-dessus du bouchon 10' de manière que le plasma seul pénètre à l'intérieur du bouchon 10'.

L'extrémité libre de la buse 40 d'injection du plasma est située à une distance (d) du bouchon 10' qui est inférieure à un centimètre (1 cm), par exemple comprise entre 0,02 cm et 0,8 cm.

La durée d'application du plasma lors de ladite troisième étape (c), de traitement, est inférieure à une seconde, par exemple comprise entre 0,3 s et 0,6 s.

En appliquant le procédé de traitement selon l'invention à des préformes 10 et à des bouchons 10' et, on obtient des récipients finaux comportant une concentration globale en peroxyde d'hydrogène qui est avantageusement réduite.

Dans le procédé de traitement d'au moins un corps creux, préforme ou bouchon, suivant les exemples d'application décrits précédemment, on recherche à tout le moins à réduire la concentration en résidus de peroxyde d'hydrogène à l'intérieur dudit corps creux, ladite réduction pouvant être obtenue en traitant avec le plasma tout ou une partie seulement de l'intérieur du corps creux.

Le plasma est donc susceptible d'être appliqué sur une partie seulement de la surface 12 interne d'une préforme 10, par exemple préférentiellement au niveau de son col 18 et/ou de son fond 16.

## Revendications

1. Procédé de fabrication de récipients à partir d'au moins une préforme (10) en matière thermoplastique, ledit procédé comportant une étape de transformation de ladite préforme (10) en récipient consistant à utiliser une unité de moulage de récipients formés par soufflage ou étirage-soufflage, et comportant successivement au moins les étapes suivantes :
- une première étape, consistant à traiter au moins l'intérieur de ladite préforme (10) au moyen de peroxyde d'hydrogène (H₂O₂) ;
- une seconde étape, consistant à chauffer un corps (14) de ladite préforme (10) à une température de moulage pour permettre sa transformation ultérieure en récipient;
**caractérisé en ce qu'il** comprend une troisième étape, antérieure à ladite étape de transformation, laquelle troisième étape consistant à traiter ladite préforme (10) avec du plasma pour réduire la concentration en résidus de peroxyde d'hydrogène (H₂O₂).

2. Procédé de fabrication selon la revendication 1, **caractérisé en ce que**, lors de la troisième étape, le plasma est émis par au moins une buse (40) qui est agencée de manière que le plasma seul pénètre à l'intérieur de la préforme (10).

3. Procédé de fabrication selon la revendication 1 ou 2, **caractérisé en ce que** la première étape (a), de traitement, consiste à déposer par condensation un film de peroxyde d'hydrogène au moins à l'intérieur de la préforme(10) en vue de sa décontamination.

4. Procédé de traitement selon l'une quelconque des revendications, **caractérisé en ce que** la deuxième étape (b), de chauffage, consiste à chauffer la préforme (10), dans lequel se trouve du peroxyde d'hydrogène, à une température déterminée qui est supérieure à la température d'activation du peroxyde d'hydrogène.

5. Procédé de fabrication selon l'une quelconque des revendications précédentes, **caractérisé en ce que** la deuxième étape (b), de chauffage de la préforme (10), est réalisée par des moyens (34, 46) de chauffage, tels que des moyens de chauffage à rayonnement infrarouge (IR), des moyens de chauffage de type laser ou par micro-ondes, des moyens de chauffage par air chaud.

6. Procédé de fabrication selon l'une des revendications précédentes, **caractérisé en ce que** l'extrémité libre de la buse (40) d'injection du plasma est située à une distance (d) de la préforme (10) qui est inférieure à un centimètre (1 cm), par exemple comprise entre 0,02 cm et 0,8 cm.

7. Procédé de fabrication selon l'une quelconque des revendications précédentes, **caractérisé en ce que** la durée d'application du plasma lors de la troisième étape (c), de traitement, est inférieure à une seconde (1 s), par exemple comprise entre 0,3 s et 0,6 s.

8. Procédé de fabrication selon l'une des revendications précédentes, comprenant en outre une phase de traitement d'au moins un bouchon (10') en matière thermoplastique destiné au récipient formé, ledit procédé comportant successivement au moins les étapes suivantes :
(a) une première étape, consistant à traiter au moins l'intérieur du bouchon (10') au moyen de peroxyde d'hydrogène (H₂O₂) ;
(b) une seconde étape, consistant à chauffer ledit bouchon (10') ;
(c) une troisième étape, consistant à traiter ledit bouchon (10') avec du plasma pour réduire la concentration en résidus de peroxyde d'hydrogène (H₂O₂).

9. Installation (100) pour la mise en œuvre du procédé de fabrication de récipients à partir de préformes (10) en matière thermoplastique, ladite installation (100) comportant au moins :
- une unité (102) de traitement de l'intérieur au moins des préformes (10) par application de peroxyde d'hydrogène ;
- une unité (104) de chauffage des préformes (10) ;
- une unité (106) de moulage de récipients formés par soufflage ou par étirage-soufflage à partir des préformes (10) chaudes ;
**caractérisée en ce que** l'installation (100) comporte au moins une unité (110) de traitement par plasma des préformes (10) décontaminées qui est agencée en amont de l'unité (106) de moulage pour réduire la concentration en résidus de peroxyde d'hydrogène à l'intérieur des préformes (10).

10. Installation (100) pour la mise en œuvre du procédé de fabrication de récipients selon la revendication 9, **caractérisée en ce que** l'unité (102) de traitement par application de peroxyde d'hydrogène est agencée en amont de l'unité (104) de chauffage des préformes et **en ce que** l'unité (110) de traitement par plasma est agencée entre l'unité (104) de chauffage et l'unité (106) de moulage, afin que le traitement des préformes par plasma soit effectué après qu'elles aient été préalablement décontaminées par le peroxyde d'hydrogène dans l'unité (102) de traitement correspondante.

## Patentansprüche

1. Verfahren zur Herstellung von Behältern aus mindestens einem Vorformling (10) aus thermoplastischem Material, wobei das Verfahren einen Schritt des Umwandelns des Vorformlings (10) in einen Behälter umfasst, der darin besteht, eine Einheit zum Formen von durch Blasen oder Streckblasen gebildeten Behältern zu verwenden, und nacheinander mindestens die folgenden Schritte umfasst:
- einen ersten Schritt, der darin besteht, mindestens das Innere des Vorformlings (10) mit Wasserstoffperoxid (H₂O₂) zu behandeln;
- einen zweiten Schritt, der darin besteht, einen Körper (14) des Vorformlings (10) auf eine Formtemperatur zu erhitzen, um seine nachfolgende Umwandlung in einen Behälter zu ermöglichen;
**dadurch gekennzeichnet, dass** es einen dritten Schritt beinhaltet, der vor dem Umwandlungsschritt erfolgt, wobei der dritte Schritt darin besteht, den Vorformling (10) mit Plasma zu behandeln, um die Konzentration von Wasserstoffperoxidrückständen (H₂O₂) zu reduzieren.

2. Herstellungsverfahren nach Anspruch 1, **dadurch gekennzeichnet, dass** während des dritten Schritts das Plasma durch mindestens eine Düse (40) ausgestoßen wird, die so angeordnet ist, dass nur das Plasma in das Innere des Vorformlings (10) eintritt.

3. Herstellungsverfahren nach Anspruch 1 oder 2, **dadurch gekennzeichnet, dass** der erste Schritt (a) des Behandelns darin besteht, durch Kondensation einen Wasserstoffperoxidfilm mindestens im Inneren des Vorformlings (10) abzuscheiden, um diesen zu dekontaminieren.

4. Behandlungsverfahren nach einem der Ansprüche, **dadurch gekennzeichnet, dass** der zweite Schritt (b) des Erhitzens darin besteht, den Vorformling (10), in dem sich Wasserstoffperoxid befindet, auf eine bestimmte Temperatur zu erhitzen, die höher als die Aktivierungstemperatur des Wasserstoffperoxids ist.

5. Herstellungsverfahren nach einem der vorhergehenden Ansprüche, **dadurch gekennzeichnet, dass** der zweite Schritt (b) des Erhitzens des Vorformlings (10) durch Mittel (34, 36) zum Erhitzen durchgeführt wird, wie etwa Mittel zum Erhitzen durch Infrarotstrahlung (IR), Mittel zum Erhitzen mittels Laser oder durch Mikrowellen, Mittel zum Erhitzen durch heiße Luft.

6. Herstellungsverfahren nach einem der vorhergehenden Ansprüche, **dadurch gekennzeichnet, dass** sich das freie Ende der Düse (40) zum Einspritzen des Plasmas in einem Abstand (d) von dem Vorformling (10) befindet, der kleiner als ein Zentimeter (1 cm) ist, beispielsweise zwischen 0,02 cm und 0,8 cm liegt.

7. Herstellungsverfahren nach einem der vorhergehenden Ansprüche, **dadurch gekennzeichnet, dass** die Anwendungsdauer des Plasmas während des dritten Schritts (c) des Behandelns kleiner als eine Sekunde (1 s) ist, beispielsweise zwischen 0,3 s und 0,6 s liegt.

8. Herstellungsverfahren nach einem der vorhergehenden Ansprüche, ferner beinhaltend eine Phase des Behandelns mindestens eines Deckels (10') aus thermoplastischem Material, der für den gebildeten Behälter bestimmt ist, wobei das Verfahren nacheinander mindestens die folgenden Schritte umfasst:
(a) einen ersten Schritt, der darin besteht, mindestens das Innere des Deckels (10') mit Wasserstoffperoxid (H₂O₂) zu behandeln;
(b) einen zweiten Schritt, der darin besteht, den Deckel (10') zu erhitzen;
(c) einen dritten Schritt, der darin besteht, den Deckel (10') mit Plasma zu behandeln, um die Konzentration von Wasserstoffperoxidrückständen (H₂O₂) zu reduzieren.

9. Anlage (100) zur Umsetzung des Verfahrens zur Herstellung von Behältern aus Vorformlingen (10) aus thermoplastischem Material, wobei die Anlage (100) mindestens Folgendes umfasst:
- eine Einheit (102) zum Behandeln des Inneren mindestens der Vorformlinge (10) mittels Anwendung von Wasserstoffperoxid;
- eine Einheit (104) zum Erhitzen der Vorformlinge (10);
- eine Einheit (106) zum Formen von durch Blasen oder Streckblasen gebildeten Behältern aus den heißen Vorformlingen (10);
**dadurch gekennzeichnet, dass** die Anlage (100) mindestens eine Einheit (110) zum Behandeln der dekontaminierten Vorformlinge (10) mittels Plasma umfasst, die vor der Einheit (106) zum Formen angeordnet ist, um die Konzentration von Wasserstoffperoxidrückständen im Inneren der Vorformlinge (10) zu reduzieren.

10. Anlage (100) zur Umsetzung des Verfahrens zur Herstellung von Behältern nach Anspruch 9, **dadurch gekennzeichnet, dass** die Einheit (102) zum Behandeln mittels Anwendung von Wasserstoffperoxid vor der Einheit (104) zum Erhitzen der Vorformlinge angeordnet ist und dass die Einheit (110) zum Behandeln mittels Plasma zwischen der Einheit (104) zum Erhitzen und der Einheit (106) zum Formen angeordnet ist, damit die Behandlung der Vorformlinge mittels Plasma erfolgt, nachdem diese zuvor in der entsprechenden Behandlungseinheit (102) durch das Wasserstoffperoxid dekontaminiert worden sind.

## Claims

1. Process for manufacturing containers from at least one preform (10) made of thermoplastic material, said process comprising a step of transforming said preform (10) into a container consisting in using a unit for moulding formed containers by blow moulding or stretch-blow moulding, and successively comprising the following steps:
- a first step, consisting in treating at least the inside of said preform (10) using hydrogen peroxide (H₂O₂) ;
- a second step, consisting in heating a body (14) of said preform (10) at a moulding temperature to enable the subsequent transformation thereof into a container;
**characterized in that** it comprises a third step, prior to the transformation step, which third step consists in treating said preform (10) with plasma to reduce the concentration of hydrogen peroxide (H₂O₂) residues.

2. Manufacturing process according to Claim 1, **characterized in that**, during the third step, the plasma is emitted by at least one nozzle (40) which is arranged so that the plasma alone penetrates inside the preform (10) .

3. Manufacturing process according to Claim 1 or 2, **characterized in that** the first step (a), of treatment, consists in depositing, by condensation, a film of hydrogen peroxide at least inside the preform (10) with a view to the decontamination thereof.

4. Treatment process according to any one of the claims, **characterized in that** the second step (b), of heating, consists in heating the preform (10), in which hydrogen peroxide is found, at a given temperature which is above the activation temperature of the hydrogen peroxide.

5. Manufacturing process according to any one of the preceding claims, **characterized in that** the second step (b), of heating the preform (10), is carried out by heating means (34, 46), such as infrared (IR) radiation heating means, laser or microwave heating means, or hot air heating means.

6. Manufacturing process according to one of the preceding claims, **characterized in that** the free end of the plasma injection nozzle (40) is located at a distance (d) from the preform (10) which is less than one centimetre (1 cm), for example between 0.02 cm and 0.8 cm.

7. Manufacturing process according to any one of the preceding claims, **characterized in that** the plasma application time during the third step (c), of treatment, is less than one second (1 s), for example between 0.3 s and 0.6 s.

8. Manufacturing process according to one of the preceding claims, further comprising a phase of treating at least one cap (10') made of thermoplastic material intended for the formed container, said process successively comprising at least the following steps:
(a) a first step, consisting in treating at least the inside of the cap (10') using hydrogen peroxide (H₂O₂) ;
(b) a second step, consisting in heating said cap (10');
(c) a third step, consisting in treating said cap (10') with plasma to reduce the concentration of hydrogen peroxide (H₂O₂) residues.

9. Equipment (100) for the implementation of the process for manufacturing containers from preforms (10) made of thermoplastic material, said equipment (100) comprising at least:
- a unit (102) for treating the inside at least of the preforms (10) by application of hydrogen peroxide;
- a unit (104) for heating preforms (10);
- a unit (106) for moulding formed containers by blow moulding or stretch-blow moulding from hot preforms (10) ;
**characterized in that** the equipment (100) comprises at least one unit (110) for plasma treatment of the decontaminated preforms (10) which is arranged upstream of the moulding unit (106) to reduce the concentration of hydrogen peroxide residues inside the preforms (10).

10. Equipment (100) for the implementation of the process for manufacturing containers according to Claim 9, **characterized in that** the unit (102) for treatment by application of hydrogen peroxide is arranged upstream of the unit (104) for heating preforms and **in that** the unit (110) for plasma treatment is arranged between the heating unit (104) and the moulding unit (106), so that the plasma treatment of the preforms is carried out after they have been previously decontaminated by the hydrogen peroxide in the corresponding treatment unit (102).
